# EUROPÄISCHE PATENTSCHRIFT

(11) **EP 2 384 738 B1**
(45) Veröffentlichungstag und Bekanntmachung des Hinweises auf die Patenterteilung: **01.11.2017**
(21) Anmeldenummer: 10015512.6
(22) Anmeldetag: 10.12.2010
(51) Int. Cl.: A61K 8/37, A61K 8/42, A61Q 19/00

(54) **Verwendung von Salicylsäureestern zur Geruchsmaskierung von Abbauprodukten des Harnstoffs**
Use of salicylic acid esters for masking the odour of degradation products from urea
Utilisation d'esters salicyliques pour le masquage d'odeur de produits de dégradation de l'urée

(30) Priorität: 18.01.2010 DE 102010004937
(43) Veröffentlichungstag der Anmeldung: 09.11.2011
(73) Patentinhaber: Beiersdorf AG, 20253 Hamburg (DE)
(72) Erfinder: Filbry, Alexander, Dr., 22459 Hamburg (DE); Zelle, Dagmar, 21640 Bliedersdorf (DE); Kröpke, Rainer, 22869 Schenefeld (DE)
(74) Vertreter: Wilke, Jochen

(56) Entgegenhaltungen:
- EP-A2- 0 927 554
- DE-A1-102004 002 170
- DE-A1-102004 052 833

## Beschreibung

Riechen ist einer der fünf Sinne des Menschen. Geruchsstoffe, leicht flüchtige Substanzen, werden dabei von Geruchsrezeptoren in der Nasenschleimhaut gebunden. Über eine Signalkaskade wird der Reiz auf die Nervenzellen übertragen und ins Gehirn weitergeleitet. Dort entsteht das eigentliche Geruchsempfinden. Angenehme Gerüche werden von unangenehmen Gerüchen unterschieden.

Harnstoff gilt in ca. 2%igen Zubereitungen als *granulationsfördernd* (als Granulationsgewebe bezeichnet man das typische junge, gefäßreiche Bindegewebe, das bei der Wund- und Geschwürsheilung bzw. bei chronischen Entzündungen gebildet wird und sich nach einiger Zeit in Narbengewebe umbildet).

In höheren Konzentrationen (5-10%) nutzt der Hautarzt den *hygroskopischen* (wasseranziehenden) Effekt des Harnstoffs aus, z.B. bei der Behandlung der Ichthyosis ("Fischschuppenkrankheit"), der Schuppenflechte (Psoriasis) und der Neurodermitis (atopisches Ekzem). *Hygroskopische* Substanzen, in erster Linie Harnstoff, halten als natürliche Feuchthaltefaktoren die Feuchtigkeit in der Hornhaut zurück. Sinkt der Wassergehalt in der Hornschicht (verursacht durch einen Harnstoffmangel) unter 15%, z.B. bei der Neurodermitis atopica, wird die Haut trocken, spröde und rissig. Ähnlich wie beim atopischen Ekzem fehlt auch bei der Schuppenflechte Harnstoff in der Epidermis. Die Folge ist eine starke Austrocknung der befallenen Hautflächen.

In kosmetischen Hautpräparaten verwendet man Harnstoff als in der Haut feuchtigkeitsbindende Substanz (1 bis 2%ig). Eine 10%ige wäßrige Lösung von Harnstoff ist bakterientötend. Schließlich wirkt Harnstoff - was man sich sowohl in Pharmazie wie auch Kosmetik zunutze machen kann - keratolytisch bzw. keratoplastisch (erweichender Effekt auf das Keratin der Haut) sowie juckreizmildernd. Durch seine polare Struktur hält er das Wasser in der Haut fest und bewahrt ihr dadurch Glätte und Geschmeidigkeit.

Die Wirkung des Wirkstoffs Allantoin beruht ebenfalls auf Harnstoff, da dieser aus dem Allantoin abgespalten wird.

Problematisch ist aber, daß Harnstoff in kosmetischen und dermatologischen Zubereitungen einem allmählichen Abbauprozeß unterworfen ist, bei welchem Ammoniak entsteht, welcher für sich genommen bereits äußerst unangenehm riecht. Ammoniak kann aber auch bei parfümierten Produkten mit den Duftbestandteilen wechselwirken und zusätzlich unangenehme Nebengerüche erzeugen. EP 0 927 554 befasst sich mit der Aufgabe, den Ammoniak-Geruch, der durch den Abbau von Harnstoff entsteht, in kosmetischen Zubereitungen zu beseitigen. Diese Aufgabe wird in EP 0 927 554 durch die Verwendung von einem Puffersystem gelöst. Aufgabe der vorliegenden Erfindung war es, die Nachteile des Standes der Technik zu beseitigen.

Überraschenderweise wird die Aufgabe gelöst durch die Verwendung einer oder mehrerer Substanzen, gewählt aus der Gruppe 4-Isopropylbenzylsalicylat, 2-Ethylhexylsalicylat, Homomenthylsalicylat zur Geruchsmaskierung von Ammoniak als Abbauprodukt des Harnstoffs in kosmetischen oder dermatologischen Zubereitungen.

Die erfindungsgemäß verwendeten Salicylsäurederivate sind gebräuchliche UV-Filter. Sie zeichnen sich durch folgende Strukturen aus:

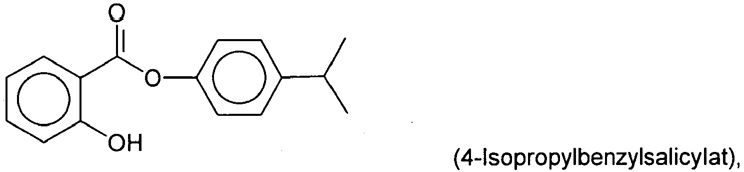

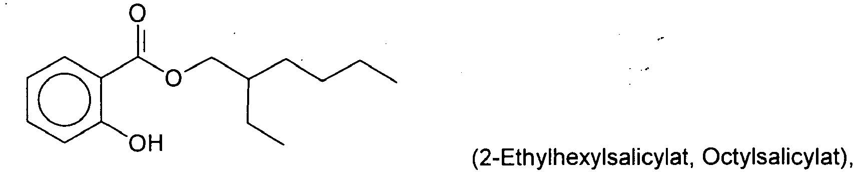

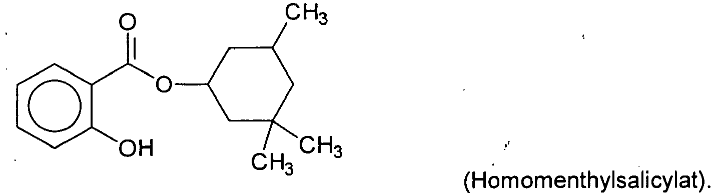

Vorteilhaft wird das Gewichtsverhältnis aus Harnstoff einerseits und der Gesamtmenge einer oder mehrerer Substanzen, gewählt aus der Gruppe 4-Isopropylbenzylsalicylat, 2-Ethylhexylsalicylat, Homomenthylsalicylat andererseits aus dem Bereich von 100 : 1 bis 1 zu 100, bevorzugt 10 : 1 bis 1 zu 10, insbesondere bevorzugt 2 : 1 bis 1 : 2 gewählt.

Kosmetische oder dermatologische Zubereitungen gemäß der Erfindung enthalten vorteilhaft 0,01 bis 60 Gew.-%, bevorzugt 0,1 bis 25 Gew.-%, insbesondere bevorzugt 1 bis 10 Gew.-% an Harnstoff, bezogen auf das Gesamtgewicht der Zubereitungen.

Kosmetische oder dermatologische Zubereitungen gemäß der Erfindung enthalten vorteilhaft 0,0001 bis 15 Gew.-%, bevorzugt 0,01 bis 10 Gew.-%, insbesondere bevorzugt 0,05 bis 2,5 Gew.-% an einer oder mehreren, Substanzen, gewählt aus der Gruppe 4-Isopropylbenzylsalicylat, 2-Ethylhexylsalicylat, Homomenthylsalicylat, bezogen auf das Gesamtgewicht der Zubereitungen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Die kosmetischen und dermatologischen Zubereitungen gemäß der Erfindung können kosmetische oder galenische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im Allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, Glycerylglucose, NMF-Komplex, Ceramide, Cholesterol, Sterol oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl-oder -monobutylether, Propylenglykolmonomethyl, -monoethyl- oder -monobutylether, Diethylenglykolmonomethyl- oder-monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl-oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Zubereitungen erfindungsgemäß weitere Substanzen enthalten, die UV-Strahlung im UVB-Bereich absorbieren, wobei die Gesamtmenge der Filtersubstanzen z.B. 0,1 Gew.% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Rezepturbeispiele** | **netero_1118** | **netero_1119** | **netero_1120** |
|---|---|---|---|
| Cetylstearylalcohol | 2,5000 | 2,5000 | 2,5000 |
| Glycerylstearat SE | 1,0000 | 1,0000 | 1,0000 |
| Wasser + Na₃HEDTA | 1,0000 | 1,0000 | 1,0000 |
| BHT | 0,0500 | 0,0500 | 0,0500 |
| Phenoxyethanol | 0,5000 | 0,5000 | 0,5000 |
| Dimethicon | 2,0000 | 2,0000 | 2,0000 |
| **Harnstoff** | - | 5,0000 | 5,0000 |
| Hydrogenierte Kokosfettsäureglyceride | 2,0000 | 2,0000 | 2,0000 |
| Butylmethoxydibenzoylmethan | 3,5000 | 3,5000 | 3,5000 |
| Milchsäure | 0,2000 | 0,2000 | 0,2000 |
| Natriumlactat | 4,2000 | 4,2000 | 4,2000 |
| Wasser + NaOH | 0,0640 | 0,0640 | 0,0640 |
| **Ethylhexylsalicylat** | 2,0000 | - | 2,0000 |
| Cetylstearylalcohol + Natriumcetylstearylsulfat | 3,0000 | 3,0000 | 3,0000 |
| Butyrospermum Parkii Butter | 2,0000 | 2,0000 | 2,0000 |
| Acrylates/C10-30 Alkyl Acrylate Crosspolymer | 0,2000 | 0,2000 | 0,2000 |
| Natriumhyaluronat | 0,3000 | 0,3000 | 0,3000 |
| C18-36 Fettsäuretriglyceride | 0,5000 | 0,5000 | 0,5000 |
| Ethylhexylglycerin | 0,5000 | 0,5000 | 0,5000 |
| Polyglyceryl-3 Methylglucosedistearat | 0,1000 | 0,1000 | 0,1000 |
| Arginin Hydrochlorid | 1,2000 | 1,2000 | 1,2000 |
| Octocrylen | 3,0000 | 3,0000 | 3,0000 |
| Chondrus Crispus | 0,2500 | 0,2500 | 0,2500 |
| Bis-Ethylhexyloxyphenolmethoxyphenyltriazin | 1,5000 | 1,5000 | 1,5000 |
| Butylenglycoldicaprylat/Dicaprat | 2,0000 | 2,0000 | 2,0000 |
| Glycine Soja | 0,1000 | 0,1000 | 0,1000 |
| Polymethylsilsesquioxan | 1,0000 | 1,0000 | 1,0000 |
| 1,2-Hexandiol | 0,5000 | 0,5000 | 0,5000 |
| Wasser | ad 100,0000 | | |

Die Zubereitungen werden in einer Kunststoffflasche oder Glastiegel (je nach Viskosität) abgefüllt und bei folgenden Lagerbedingungen eingelagert:
+ 6 °C (Referenz), Raumtemperatur, Brutschrank 40°C und Licht (Südfenster im Gebäude).

Die Testzeitpunkte sind nach 2, 4 und 6 Monaten. Die Proben werden den jeweiligen Lokali-täten entnommen und erst nach 24 Stunden bei Raumtemperatur mit einem ausgebildeten Parfümeur beurteilt.

Ergebnisse:

| **Ansatz** | **Packmittel/parfum** | **°C** | **2 Monate** | **4 Monate** | **6 Monate** |
|---|---|---|---|---|---|
| **NETERO 1118** | ohne Harnstoff | +6 | **100** | **95** | **95** |
| **NETERO 1118** | ohne Harnstoff | RT | **95** | **70** | **70** |
| **NETERO 1118** | ohne Harnstoff | 40 | **95** | **90** | **90** |
| **NETERO 1118** | ohne Harnstoff | Licht | **80** | **70** | **70** |
| **NETERO 1119** | ohne Octylsalicylat | +6 | **100** | **80 starker Käsefuß** | **60 starker Käsefuß** |
| **NETERO 1119** | ohne Octylsalicylat | RT | **80** | **70** | 50 |
| **NETERO 1119** | ohne Octylsalicylat | 40 | **90** | **85** | **40 leichter Ammoniakgeruch** |
| **NETERO 1119** | ohne Octylsalicylat | Licht | **70** | **65** | **50** |
| **NETERO 1120** Vergleich | mit Harnstoff/Octylsalicylat | +6 | **100** | **100** | **100** |
| **NETERO 1120** Vergleich | mit Harnstoff/Octylsalicylat | RT | **95** | **80** | **80** |
| **NETERO 1120** Vergleich | mit Harnstoff/Octylsalicylat | 40 | **95** | **90** | **85 Käsefuß/ Ammoniakgeruch deutlich vermindert** |
| **NETERO 1120** Vergleich | mit Harnstoff/Octylsalicylat | Licht | **80** | **80** | **80** |

Netero1120, das erfindungsgemäße Präparat zeigt gegenüber den Vergleichspräparaten eine deutlich verbesserte Stabilität.

## Patentansprüche

1. Verwendung einer oder mehrerer Substanzen, gewählt aus der Gruppe 4-Isopropylbenzylsalicylat, 2-Ethylhexylsalicylat, Homomenthylsalicylat zur Geruchsmaskierung von Ammoniak als Abbauprodukt des Harnstoffs in kosmetischen oder dermatologischen Zubereitungen.

2. Verwendung nach Anspruch 1, **dadurch gekennzeichnet, dass** das Gewichtsverhältnis aus Harnstoff einerseits und der Gesamtmenge einer oder mehrerer Substanzen, gewählt aus der Gruppe 4-Isopropylbenzylsalicylat, 2-Ethylhexylsalicylat, Homomenthylsalicylat andererseits aus dem Bereich von 100 : 1 bis 1 zu 100, bevorzugt 10 : 1 bis 1 zu 10, insbesondere bevorzugt 2 : 1 bis 1 : 2 gewählt wird.

3. Verwendung nach Anspruch 1 oder 2 in kosmetischen oder dermatologischen Zubereitungen, die 0,01 bis 60 Gew.-%, bevorzugt 0,1 bis 25 Gel.-%, insbesondere bevorzugt 1 bis 10 Gew.-% an Harnstoff, bezogen auf das Gesamtgewicht der Zubereitungen.

## Claims

1. Use of one or more substances selected from the group consisting of 4-isopropylbenzyl salicylate, 2-ethylhexyl salicylate, homomenthyl salicylate for masking the odour of ammonia as degradation product of the urea in cosmetic or dermatological preparations.

2. Use according to Claim 1, **characterized in that** the weight ratio of urea, on the one hand, and the total amount of one or more substances selected from the group consisting of 4-isopropylbenzyl salicylate, 2-ethylhexyl salicylate, homomenthyl salicylate, on the other, is selected from the range of from 100:1 to 1 to 100, preferably from 10:1 to 1 to 10, especially preferably from 2:1 to 1:2.

3. Use according to Claim 1 or 2 in cosmetic or dermatological preparations which from 0.01 to 60% by weight, preferably from 0.1 to 25% by weight, especially preferably from 1 to 10% by weight, of urea, based on the total weight of the preparations.

## Revendications

1. Utilisation d'une ou de plusieurs substances, choisies dans le groupe constitué par le salicylate de 4-isopropylbenzyle, le salicylate de 2-éthylhexyle, le salicylate d'homo-menthyle pour masquer une odeur d'ammoniac en tant que produit de décomposition de l'urée dans des préparations cosmétiques ou dermatologiques.

2. Utilisation selon la revendication 1, **caractérisée en ce que** le rapport en poids entre l'urée d'un côté et la quantité totale d'une ou de plusieurs substances choisies dans le groupe constitué par le salicylate de 4-isopropylbenzyle, le salicylate de 2-éthylhexyle, le salicylate d'homomenthyle d'un autre côté est choisi dans la plage allant de 100:1 à 1:100, de préférence de 10:1 à 1:10, de manière particulièrement préférée de 2:1 à 1:2.

3. Utilisation selon la revendication 1 ou 2 dans des préparations cosmétiques ou dermatologiques qui 0,01 à 60 % en poids, de préférence 0,1 à 25 % en poids, de manière particulièrement préférée 1 à 10 % en poids d'urée, par rapport au poids total des préparations.
